# EUROPEAN PATENT APPLICATION

(11) **EP 3 321 659 A1**
(43) Date of publication of application: **16.05.2018**
(21) Application number: 16821226.4
(22) Date of filing: 20.06.2016
(51) Int. Cl.: G01N 21/27, G01J 3/46, G01N 33/48

(54) **CHROMOSCOPY DEVICE AND CHROMOSCOPY METHOD**

(30) Priority: 09.07.2015 JP 2015137680
(71) Applicant: Olympus Corporation, Hachioji-shi Tokyo 192-8507 (JP)
(72) Inventor: KAWANO, Yoshihiro, Hachioji-shi Tokyo 192-8507 (JP)
(74) Representative: Gunzelmann, Rainer
(86) International application number: PCT/JP2016/068303
(87) International publication number: WO 2017/006756

(57) **Abstract**

A dye measurement device (1) includes: an illumination unit (4) that radiates illumination light onto a specimen (O) stained with three or more dyes; an image acquisition unit (5) that captures transmitted light, transmitted through the specimen (O), of the illumination light radiated by the illumination unit (4), to obtain image signals; a narrow-band light generating unit (3) that is disposed in the illumination unit (4) or the image acquisition unit (5) and that generates, with a switching manner, a plurality of narrow band light beams having different narrow bands in each of two or more different wavelength bands; and an image processing unit (6) that determines an abundance ratio of the dyes through unmixing processing by using four or more of the image signals obtained when the image acquisition unit (5) captures the narrow-band light beams generated by the narrow-band light generating unit (3).

## Description

### {Technical Field}

The present invention relates to a dye measurement device and a dye measurement method.

### {Background Art}

In the related art, in order to make a pathological diagnosis, observation of a hematoxylin & eosin (H&E) stained specimen using transmission illumination is performed. Furthermore, in order to clarify subclassification of diseases that cannot be found through H&E staining alone, proteins are stained through IHC staining, or genes are stained through ISH staining. Furthermore, in order to obtain a lot of information from the same specimen, proteins are subjected to multiple staining (for example, see PTLs 1 to 3).

### {Citation List}

### {Patent Literature}

{PTL 1} US Patent No. 6403332
{PTL 2} US Patent No. 6025601
{PTL 3} Japanese Examined Patent Application, Publication No. H07-50031

### {Summary of Invention}

### {Technical Problem}

When the same specimen is subjected to multiple staining by using a plurality of dyes, because the spectra of the dyes overlap, it is necessary to acquire a spectral image of the specimen to acquire a true-dye image.

However, there is a disadvantage in which, if an ordinary monochrome camera is used to acquire a spectral image, image capturing takes time.

The present invention has been made in view of the above-described circumstances, and an object thereof is to provide a dye measurement device and a dye measurement method capable of acquiring a spectral image of a specimen in a short period of time with a simple configuration, to acquire a true-dye image.

### {Solution to Problem}

In order to achieve the above-described object, the present invention provides the following solutions.

A first aspect of the present invention is a dye measurement device including: an illumination unit that radiates illumination light onto a specimen stained with three or more dyes; an image acquisition unit that captures transmitted light, transmitted through the specimen, of the illumination light radiated by the illumination unit in order to obtain image signals; a narrow-band light generating unit that is disposed in the illumination unit or the image acquisition unit and that generates, with a switchable manner, a plurality of narrow band light beams having different narrow bands in each of two or more different wavelength bands; and an image processing unit that determines an abundance ratio of the dyes through unmixing processing by using four or more of the image signals obtained when the image acquisition unit captures the narrow-band light beams generated by the narrow-band light generating unit.

According to this aspect, the illumination unit radiates illumination light onto a specimen stained with three or more dyes, the image acquisition unit captures transmitted light transmitted through the specimen to obtain image signals, and the image processing unit processes the obtained image signals, thereby determining the abundance ratio of the dyes. Specifically, when the narrow-band light generating unit is provided in the illumination unit, the narrow-band light generating unit generates, with a switching manner, a plurality of narrow band light beams in each of two or more different wavelength bands and radiates them onto the specimen, and thus, the image acquisition unit captures transmitted light beams in four or more narrow bands and obtains four or more image signals.

On the other hand, when the narrow-band light generating unit is provided in the image acquisition unit, four or more narrow-band light beams are cut out from transmitted light beams obtained by radiating broadband light including two or more wavelength bands and are captured, and thus, the image acquisition unit captures transmitted light beams in four or more narrow bands and obtains four or more image signals.

Then, on the basis of the obtained four or more image signals, the abundance ratio of three or more dyes can be determined, for each pixel, through the unmixing processing. Accordingly, a spectral image of the specimen can be acquired in a short period of time with a simple configuration, to acquire a true-dye image.

A second aspect of the present invention is a dye measurement method including: a first illumination step of radiating first illumination light onto a specimen stained with three or more dyes; a first image-acquisition step of capturing transmitted light, transmitted through the specimen, of the first illumination light radiated in the first illumination step, to obtain image signals; a second illumination step of radiating second illumination light onto the specimen; a second image-acquisition step of capturing transmitted light, transmitted through the specimen, of the second illumination light radiated in the second illumination step, the transmitted light having a different wavelength from that in the first image-acquisition step, to obtain image signals; and an image processing step of determining an abundance ratio of the dyes through unmixing processing by using four or more of the image signals obtained in the first image-acquisition step and the second image-acquisition step.

According to this aspect, the first illumination light is radiated in the first illumination step; transmitted light, transmitted through the specimen, of the first illumination light is captured, thus obtaining image signals in the first image-acquisition step; the second illumination light is radiated in the second illumination step; and transmitted light, transmitted through the specimen, of the second illumination light is captured, thus obtaining image signals in the second image-acquisition step. Because the wavelength of the transmitted light captured in the second image-acquisition step differs from the wavelength of the transmitted light captured in the first image-acquisition step, four or more image signals can be easily obtained through two rounds of capturing. Then, unmixing processing is performed in the image processing step, thereby making it possible to easily determine the abundance ratio of dyes for each pixel.

In the above-described aspect, in the first image-acquisition step and the second image-acquisition step, the transmitted light may be captured by a sensor having a sensitivity to two or more different wavelength bands; the first illumination light may include two or more narrow band light beams respectively included in the wavelength bands; and the second illumination light may include one or more narrow band light beams respectively included in the wavelength bands, the narrow band light beams each having a different wavelength from those in the first illumination light.

By doing so, light beams in two or more narrow bands are radiated onto the specimen in the first illumination step, and transmitted light beams of the respective narrow-band light beams are captured, thus generating image signals in the first image-acquisition step. Furthermore, a light beam in one or more narrow bands different from those in the first illumination step is radiated onto the specimen in the second illumination step, and transmitted light of the narrow-band light beam is captured, thus generating an image signal in the second image-acquisition step. Accordingly, it is possible to easily obtain four or more image signals through two rounds of capturing and to easily determine, for each pixel, the abundance ratio of the dyes in the image processing step.

Furthermore, in the above-described aspect, the first illumination light and the second illumination light may include the nattow band light beams in four or more narrow bands in total; and in the first image-acquisition step and the second image-acquisition step, a sensor having a sensitivity to two or more different wavelength bands may capture four or more narrow band narrow light beams in total, switching the wavelengths of transmitted light beams to be captured.

By doing so, light beams in four or more different narrow bands in total are radiated onto the specimen two times in the first illumination step and the second illumination step, the wavelengths of transmitted light beams to be captured are switched, and a sensor having sensitivities to two or more different wavelength bands captures the light beams in four or more different narrow bands in total. Accordingly, it is possible to easily obtain four or more image signals through two rounds of capturing and to easily determine, for each pixel, the abundance ratio of the dyes in the image processing step.

Furthermore, in the above-described aspect, the image processing step may include: a spectral-image generating step of generating a spectral image from four or more of the image signals; a spectrum generating step of generating a transmitted-light spectrum of the specimen for each pixel on the basis of the generated spectral image; and an unmixing step of performing unmixing processing by using the generated transmitted-light spectrum and absorption spectra of the respective dyes that is stored in advance.

Furthermore, in the above-described aspect, the image processing step may include a region selecting step of selecting a target region on the basis of the spectral image generated in the spectral-image generating step; and the spectrum generating step and the unmixing step may be performed for the target region selected in the region selecting step.

By doing so, the spectrum generating step and the unmixing step are performed only for a target region selected in the region selecting step, thus reducing the processing time and making it possible to prevent the occurrence of noise caused by including, in unmixing processing, signals in unnecessary regions.

### {Advantageous Effects of Invention}

According to the present invention, an advantageous effect is afforded in that it is possible to acquire a spectral image of a specimen in a short period of time with a simple configuration, to acquire a true-dye image.

### {Brief Description of Drawings}

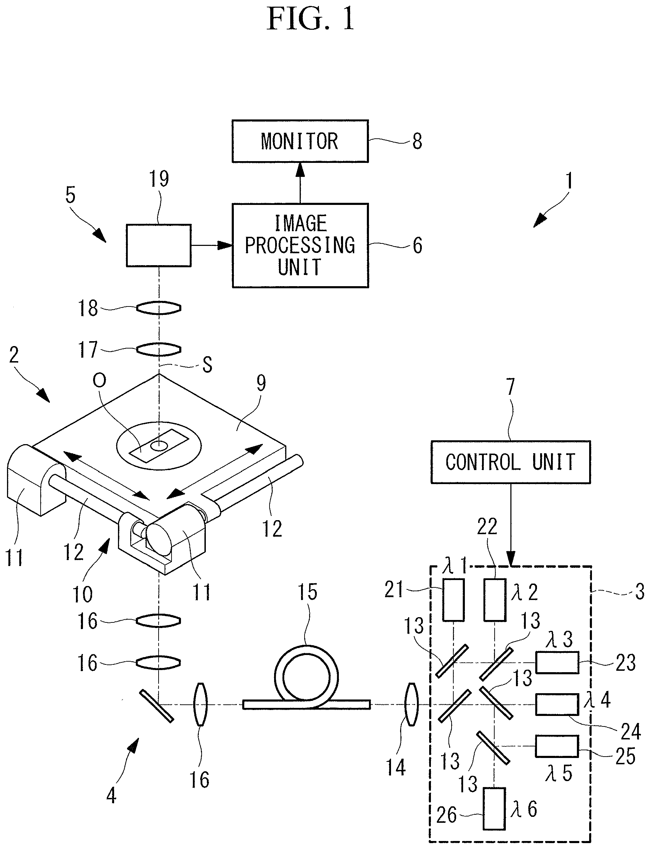

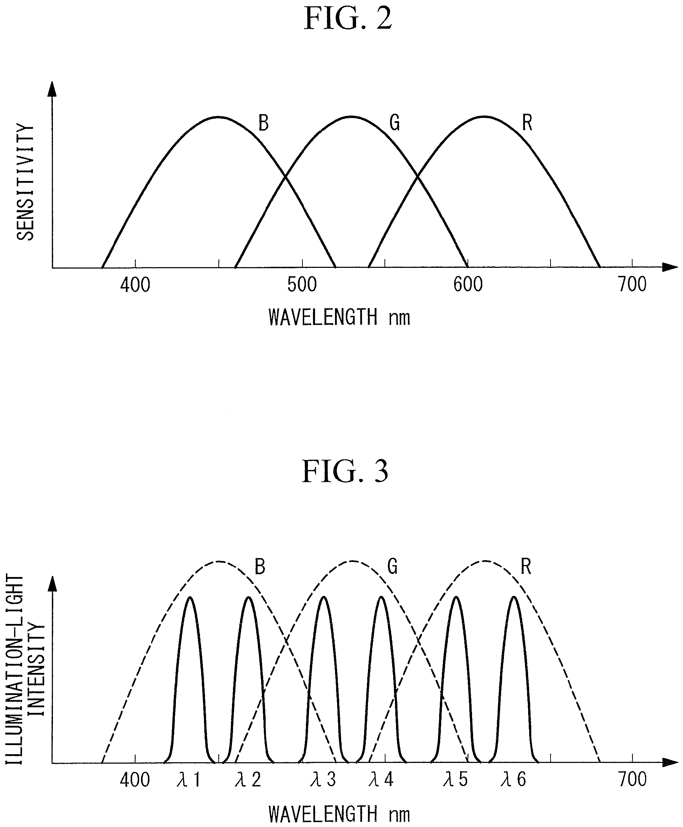

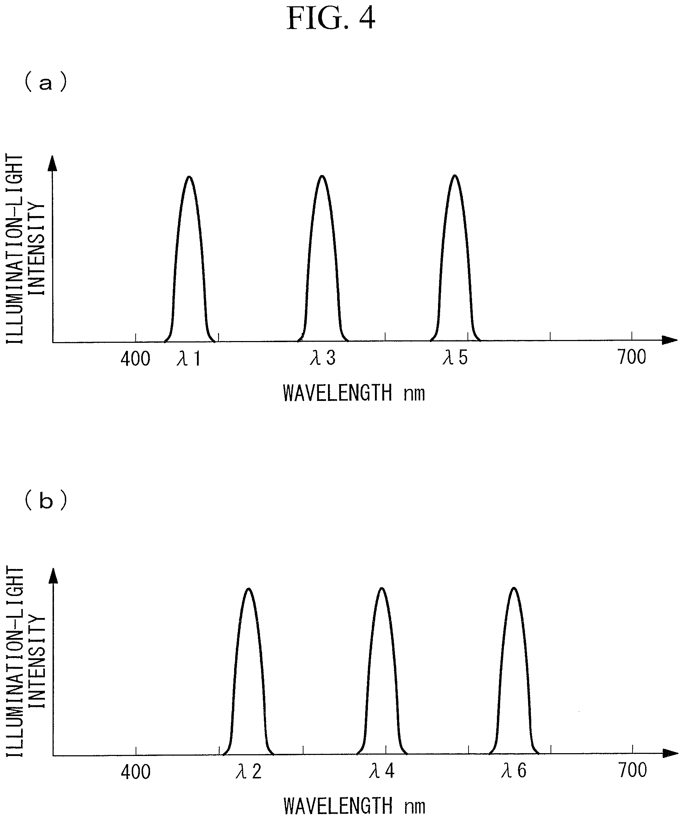

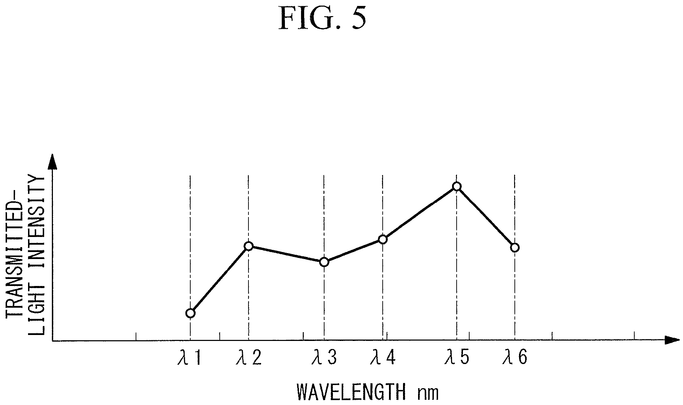

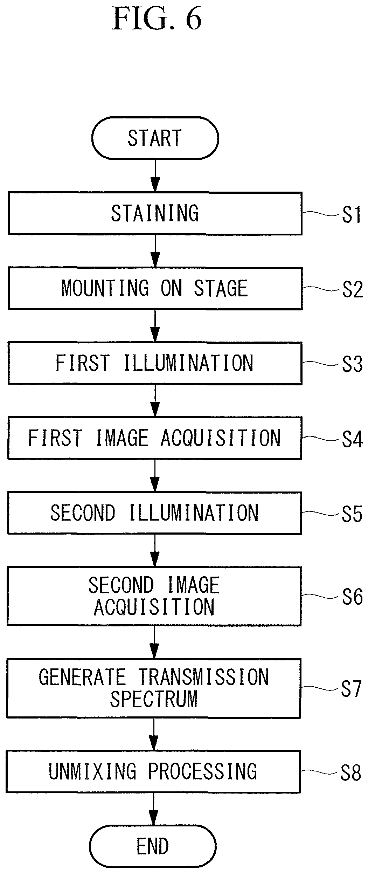

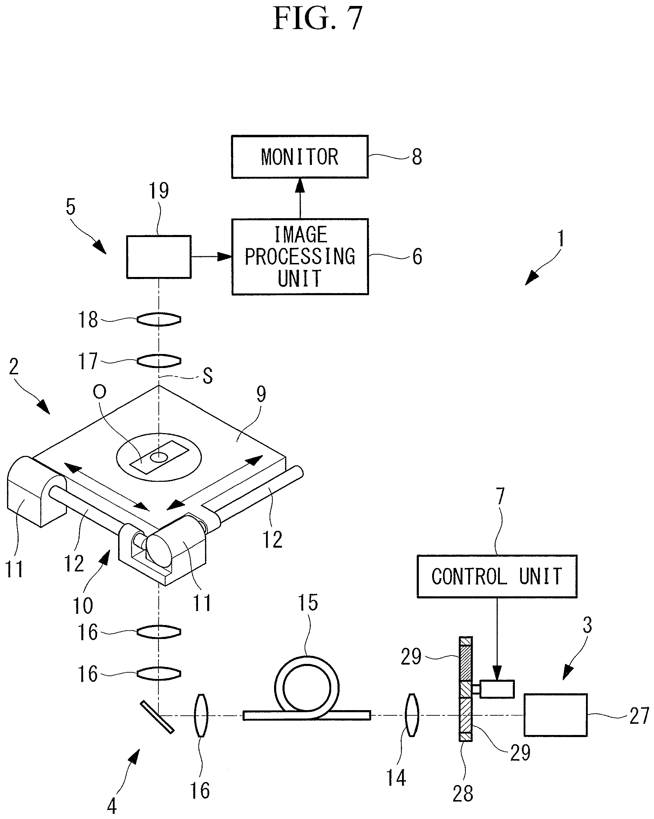

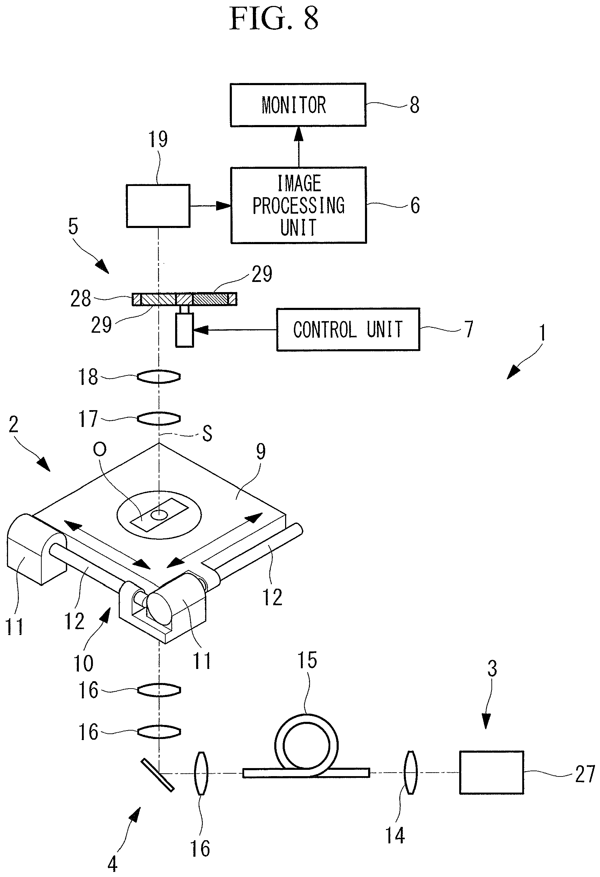

{Fig. 1} Fig. 1 is a view showing an overall configuration of a dye measurement device according to an embodiment of the present invention.
{Fig. 2} Fig. 2 is a view showing a sensitivity characteristic of a color image-acquisition element provided in the dye measurement device shown in Fig. 1.
{Fig. 3} Fig. 3 is a view showing an illumination-light intensity characteristic of a light source unit provided in the dye measurement device shown in Fig. 1.
{Fig. 4} Fig. 4 includes views showing light intensity characteristics of (a) first illumination light and (b) second illumination light emitted by the light source unit provided in the dye measurement device shown in Fig. 1.
{Fig. 5} Fig. 5 is a view showing an example transmitted-light spectrum obtained by the dye measurement device shown in Fig. 1.
{Fig. 6} Fig. 6 is a flowchart showing a dye measurement method according to the aforementioned embodiment of the present invention.
{Fig. 7} Fig. 7 is a view showing an overall configuration of a first modification of the dye measurement device shown in Fig. 1.
{Fig. 8} Fig. 8 is a view showing an overall configuration of a second modification of the dye measurement device shown in Fig. 1.

### {Description of Embodiment}

A dye measurement device 1 and a dye measurement method according to an embodiment of the present invention will be described below with reference to the drawings.

As shown in Fig. 1, the dye measurement device 1 of this embodiment is provided with: a stage 2 on which a specimen O is mounted; a light source unit (narrow-band light generating unit) 3 that generates illumination light; an illumination optical system (illumination unit) 4 that radiates illumination light generated by the light source unit 3 onto the specimen O from a position below the stage 2; an image-acquisition optical system (image acquisition unit) 5 that captures light transmitted through the specimen O, at a position above the stage 2; an image processing unit 6 that processes image signals obtained by the image-acquisition optical system 5; a control unit 7; and a monitor 8.

The stage 2 is provided with: a mounting unit 9 on which the specimen O, for example, a slide-glass specimen, is mounted and that is formed of a material capable of transmitting light from the illumination optical system 4 located therebelow or that has a through-hole formed therein; and an XY-drive mechanism 10 that moves the mounting unit 9 in two directions perpendicular to the optical axis S of the illumination optical system 4. The XY-drive mechanism 10 is, for example, a linear motion mechanism that is provided with two pairs of motors 11 and ball screws 12.

The light source unit 3 is provided with: six LEDs 21, 22, 23, 24, 25, and 26 that emit illumination light beams having different wavelengths; and dichroic mirrors 13 that merge the illumination light beams emitted by the respective LEDs 21, 22, 23, 24, 25, and 26.

The illumination optical system 4 is provided with: a coupling lens 14 that focuses the illumination light beams merged by the dichroic mirrors 13 of the light source unit 3; an optical fiber 15 that guides the illumination light focused by the coupling lens 14; and focusing lenses 16 that focus the illumination light guided by the optical fiber 15 on a predetermined area on the specimen O.

The image-acquisition optical system 5 is provided with: an objective lens 17 that collects transmitted light of the illumination light transmitted through the specimen O, at a position above the specimen O; an imaging lens 18 that forms an image of the transmitted light collected by the objective lens 17; and a color image-acquisition element 19 that acquires the image of the specimen O formed by the imaging lens 18.

The color image-acquisition element 19 has the sensitivity characteristic shown in Fig. 2. Specifically, the color image-acquisition element 19 is a so-called Bayer-array image-acquisition element in which color filters that can transmit light beams corresponding to the wavelength bands of R, G, and B are arrayed on respective pixels. Signals of the pixels, on which the respective color filters corresponding to the wavelength bands of R, G, and B are provided, are individually obtained, thereby making it possible to acquire an R image, a G image, and a B image of the specimen O.

Furthermore, in this embodiment, the six LEDs 21, 22, 23, 24, 25, and 26 of the light source unit 3 have the spectra shown in Fig. 3. Specifically, the six LEDs 21, 22, 23, 24, 25, and 26 are grouped into three groups each including two LEDs, and the two LEDs in each group have spectra in the corresponding wavelength band of R, G, or B of the color image-acquisition element 19.

The control unit 7 controls the light source unit 3 to actuate the six LEDs 21, 22, 23, 24, 25, and 26 such that, as shown in Fig. 4, three LEDs each selected from the respective groups are simultaneously turned on or off. In the example shown in Fig. 4, the control unit 7 simultaneously turns on the LEDs 21, 23, and 25 corresponding to wavelengths λ1, λ3, and λ5 shown in Fig. 4(a) and then simultaneously turns on the LEDs 22, 24, and 26 corresponding to wavelengths λ2, λ4, and λ6 shown in Fig. 4(b). When turning on one of the LEDs in the respective wavelength bands of R, G, or B, the control unit 7 turns off the other LED therein.

Accordingly, in the first capturing, the LEDs 21, 23, and 25 are simultaneously turned on, illumination light beams having the wavelengths λ1, λ3, and λ5 are simultaneously radiated onto the specimen O from the LEDs 21, 23, and 25, and transmitted light beams having the wavelengths λ1, λ3, and λ5 transmitted through the specimen O are captured by the color image-acquisition element 19 at one time. In the color image-acquisition element 19, because the color filters, which transmit the transmitted light beams in the wavelength bands of R, G, and B, respectively, including the corresponding wavelengths λ1, λ3, and λ5, are arrayed, signals of pixels on which the same color filters are disposed are collected, thereby making it possible to simultaneously and separately obtain three types of image signals corresponding to the wavelengths λ1, λ3, and λ5.

Similarly, in the next capturing, the LEDs 22, 24, and 26 are simultaneously turned on, illumination light beams having the wavelengths λ2, λ4, and λ6 are simultaneously radiated onto the specimen O from the LEDs 22, 24, and 26, and transmitted light beams having the wavelengths λ2, λ4, and λ6 transmitted through the specimen O are captured by the color image-acquisition element 19 at one time. In the color image-acquisition element 19, because the color filters, which transmit the transmitted light beams in the wavelength bands of R, G, and B, respectively, including the corresponding wavelengths λ2, λ4, and λ6, are arrayed, it is possible to simultaneously and separately obtain three types of image signals corresponding to the wavelengths λ2, λ4, and λ6.

The image processing unit 6 stores absorption spectra of dyes for staining the specimen O. In this embodiment, the specimen O is subjected to multiple staining with three dyes, for example, and the image processing unit 6 stores absorption spectrums of the three dyes.

Furthermore, the image processing unit 6 generates, for each pixel, a transmitted-light spectrum of the specimen O on the basis of six types of image signals that are obtained through the two rounds of capturing performed by the image-acquisition optical system 5. The transmitted-light spectrum can be easily generated by plotting the intensities of the six types of signals obtained at each pixel, as shown in Fig. 5, with the horizontal axis indicating the wavelength.

Then, the image processing unit 6 performs known unmixing processing on the basis of the stored absorption spectra of the three dyes and the generated transmitted-light spectrum of the specimen O, to calculate the abundance ratio of the respective dyes for each pixel.

Accordingly, it is possible to acquire an image indicating the distribution of the abundance ratios of the respective dyes.

The dye measurement method using the thus-configured dye measurement device 1 of this embodiment will be described below.

Prior to dye measurement, a standard specimen is mounted on the stage 2, and the intensities of illumination light beams to be emitted by the LEDs 21, 22, 23, 24, 25, and 26 are adjusted on the basis of the spectrum of an image acquired by the image-acquisition optical system 5 when the six LEDs 21, 22, 23, 24, 25, and 26 are simultaneously turned on. Accordingly, the intensity balance of the LEDs 21, 22, 23, 24, 25, and 26 is set such that proper white illumination light can be emitted.

As shown in Fig. 6, in the dye measurement method of this embodiment, the specimen O is stained with three dyes (Step S1), the stained specimen O is mounted on the stage 2 (Step S2), the LEDs 21, 23, and 25 are turned on, thus radiating illumination light beams having the wavelengths λ1, λ3, and λ5 (first illumination light) onto the specimen O (first illumination step S3), and the transmitted light beams transmitted through the specimen O are captured by the image-acquisition optical system 5, thus obtaining image signals (first image-acquisition step S4).

Next, the LEDs to be turned on are switched to the LEDs 22, 24, and 26, thus radiating illumination light beams having the wavelengths λ2, λ4, and λ6 (second illumination light) onto the specimen O (second illumination step S5), and the transmitted light beams transmitted through the specimen O are captured by the image-acquisition optical system 5, thus obtaining image signals (second image-acquisition step S6).

Thereafter, the abundance ratio of the dyes is determined by using the obtained six image signals (image processing step). Specifically, a spectral image is generated by using the obtained six image signals (spectral-image generating step). The image processing unit 6 generates a transmitted-light spectrum for each pixel on the basis of the generated spectral image (spectrum generating step S7). The abundance ratio of the dyes is determined through unmixing processing on the basis of the generated transmitted-light spectrum and the stored absorption spectrum of the dyes (unmixing step S8).

Accordingly, it is possible to acquire an image indicating the distribution of the abundance ratios of the respective dyes.

Specifically, according to the dye measurement device 1 and the dye measurement method of this embodiment, there is an advantage in that six types of image signals are obtained through the two rounds of capturing, thus making it possible to acquire a distribution image of three to five dyes. As a result, it is possible to acquire a spectral image of the specimen O in a short period of time with a simple configuration, to acquire a true-dye image and to detect a subclassification of a lesion in the specimen O.

Note that, in the dye measurement device 1 and the dye measurement method of this embodiment, although two illumination light beams having different wavelengths in each of the three wavelength bands of R, G, and B are radiated by switching the LEDs, illumination light beams having four or more different wavelengths need to be radiated in order to measure the abundance ratio of three dyes through unmixing processing. Therefore, for example, it is also possible to radiate illumination light beams having two different wavelengths in each of two wavelength bands by switching the LEDs such that illumination light beams having the wavelengths λ1 and λ3 are radiated as the first illumination light, and illumination light beams having the wavelengths λ2 and λ4 are radiated as the second illumination light. Furthermore, it is also possible to radiate illumination light beams having, in total, four different wavelengths such that illumination light beams having the wavelengths λ1, λ3, and λ5 are radiated as the first illumination light, and an illumination light beam having the wavelength λ2 is radiated as the second illumination light.

Furthermore, in this embodiment, although the abundance ratio of the dyes is calculated for the entire acquired image, instead of this, it is also possible to select a partial region in the acquired image (region selecting step) and to perform generation of a transmitted-light spectrum and unmixing processing only for the selected region. By doing so, there is an advantage in that it is possible to reduce the time required for the processing and to prevent the occurrence of noise caused by including, in the processing, pixels for which measurement of the distribution of the abundance ratio of the dyes is not necessary.

Furthermore, in a specimen O that has been subjected to multiple staining with six or more different dyes, in order to measure the abundance ratio of the dyes, illumination light beams having three or more different wavelengths in the three wavelength bands of R, G, and B may be generated in a switching manner. Furthermore, the number of LEDs that can simultaneously emit illumination light beams may be increased by adding another wavelength band, such as emerald, to the three colors of R, G, and B.

Furthermore, in this embodiment, although image signals of six colors are obtained through two rounds of capturing by switching the lighting of the LEDs 21, 22, 23, 24, 25, and 26, instead of this, as shown in Fig. 7, it is also possible to adopt a single white-light source 27 as a light source, to use filters 29 that can be switched by a turret 28, so as to switch the wavelength of illumination light to be radiated onto the specimen O, as in Figs. 4(a) and 4(b). Furthermore, as shown in Fig. 8, it is also possible to dispose the turret 28 in the image-acquisition optical system 5 and to switch the wavelength of transmitted light, of illumination light transmitted through the specimen O, to be captured by the color image-acquisition element 19.

Furthermore, among three dyes A, B, and C, the two dyes A and B are used as dyes for expressing different proteins, and the remaining dye C is used as a dye for detecting cell nuclei, thereby making it possible to detect the ranges of the cell nuclei by means of an image of the dye C and to measure the amounts of proteins expressed in the cell nuclei by means of images of the dyes A and B. Accordingly, it is possible to perform class determination of the levels of expression of two types of proteins for each cell nucleus.

Furthermore, as the unmixing processing, instead of a spectral unmixing method, a spectral blind unmixing method may be used.

### {Reference Signs List}

- 1: dye measurement device
- 3: light source unit (narrow-band light generating unit)
- 4: illumination optical system (illumination unit)
- 5: image-acquisition optical system (image acquisition unit)
- 6: image processing unit
- S3: first illumination step
- S4: first image-acquisition step
- S5: second illumination step
- S6: second image-acquisition step
- S7: spectrum generating step
- S8: unmixing step
- O: specimen

## Claims

1. A dye measurement device comprising:
an illumination unit that radiates illumination light onto a specimen stained with three or more dyes;
an image acquisition unit that captures transmitted light, transmitted through the specimen, of the illumination light radiated by the illumination unit in order to obtain image signals;
a narrow-band light generating unit that is disposed in the illumination unit or the image acquisition unit and that generates, with a switching manner, a plurality of narrow band light beams having different narrow bands in each of two or more different wavelength bands; and
an image processing unit that determines an abundance ratio of the dyes through unmixing processing by using four or more of the image signals obtained when the image acquisition unit captures the narrow-band light beams generated by the narrow-band light generating unit.

2. A dye measurement method comprising:
a first illumination step of radiating first illumination light onto a specimen stained with three or more dyes;
a first image-acquisition step of capturing transmitted light, transmitted through the specimen, of the first illumination light radiated in the first illumination step, to obtain image signals;
a second illumination step of radiating second illumination light onto the specimen;
a second image-acquisition step of capturing transmitted light, transmitted through the specimen, of the second illumination light radiated in the second illumination step, the transmitted light having a different wavelength from that in the first image-acquisition step, to obtain image signals; and
an image processing step of determining an abundance ratio of the dyes through unmixing processing by using four or more of the image signals obtained in the first image-acquisition step and the second image-acquisition step.

3. The dye measurement method according to claim 2,
wherein, in the first image-acquisition step and the second image-acquisition step, the transmitted light is captured by a sensor having sensitivity to two or more different wavelength bands;
the first illumination light includes two or more narrow band light beams respectively included in the wavelength bands; and
the second illumination light includes one or more narrow band light beams respectively included in the wavelength bands, the narrow band light beams each having a different wavelength from those in the first illumination light.

4. The dye measurement method according to claim 2,
wherein the first illumination light and the second illumination light include the narrow band light beams in four or more narrow bands in total; and
in the first image-acquisition step and the second image-acquisition step, a sensor having a sensitivity to two or more different wavelength bands captures four or more narrow band narrow light beams in total, switching the wavelengths of transmitted light beams to be captured.

5. The dye measurement method according to any one of claims 2 to 4, wherein the image processing step comprises:
a spectral-image generating step of generating a spectral image from four or more of the image signals;
a spectrum generating step of generating a transmitted-light spectrum of the specimen for each pixel on the basis of the generated spectral image; and
an unmixing step of performing unmixing processing by using the generated transmitted-light spectrum and absorption spectra of the respective dyes that is stored in advance.

6. The dye measurement method according to claim 5,
wherein the image processing step comprises a region selecting step of selecting a target region on the basis of the spectral image generated in the spectral-image generating step; and
the spectrum generating step and the unmixing step are performed for the target region selected in the region selecting step.
